# FASCICULE DE BREVET EUROPEEN

(11) **EP 1 053 037 B1**
(45) Date de publication et mention de la délivrance du brevet: **13.11.2002**
(21) Numéro de dépôt: 98962533.0
(22) Date de dépôt: 21.12.1998
(51) Int. Cl.: A61M 5/20, A61M 5/32

(54) **DISPOSITIF D'INJECTION AUTOMATIQUE D'UNE DOSE DE PRODUIT MEDICAMENTEUX**
VORRICHTUNG ZUR AUTOMATISCHEN INJEKTION EINER DOSIS EINES MEDIKAMENTENPRODUKTS
DEVICE FOR AUTOMATIC INJECTION OF A DOSE OF MEDICINAL PRODUCT

(30) Priorité: 04.02.1998 FR 9801298
(43) Date de publication de la demande: 22.11.2000
(73) Titulaire: SANOFI-SYNTHELABO, 75013 Paris (FR)
(72) Inventeur: BRUNEL, Marc, F-31000 Toulouse (FR)
(74) Mandataire: Barre, Philippe
(86) Numéro de dépôt international: FR9802809
(87) Numéro de publication internationale: WO99039759

(56) Documents cités:
- EP-A- 0 516 473
- EP-A- 0 518 416
- EP-A- 0 577 448
- WO-A-94/21316
- FR-A- 2 654 938
- US-A- 5 709 662

## Description

L'invention concerne un dispositif d'injection automatique d'une dose de produit médicamenteux.

Il existe à l'heure actuelle de nombreux dispositifs d'injection permettant à un patient non expérimenté de s'auto-administrer une dose de produit médicamenteux sans que ce dernier ait à enfoncer lui-même l'aiguille et actionner le piston de la seringue.

De tels dispositifs d'injection sont notamment décrits dans les brevets EP 516.473, US 3.797.489, US 3.712.301, WO 95/35126, FR 2.654.938, EP 577.448.

A l'heure actuelle, pour des raisons évidentes de sécurité, la tendance consiste à réaliser des dispositifs d'injection, tel que celui décrit dans le brevet EP 516.473, à usage unique comportant un ressort de propulsion destiné à l'injection du produit médicamenteux, et un ressort de propulsion destiné à repousser automatiquement la seringue dans le boîtier après injection. Selon cette conception, l'aiguille de la seringue ne dépasse du boîtier que durant le temps de l'injection et aucun risque de blessure avec une aiguille souillée n'est donc à redouter.

Bien que de tels dispositifs d'injection apportent un avantage notable quant à leur sécurité d'utilisation, ils présentent toutefois plusieurs inconvénients. En effet, et en premier lieu, du fait des tolérances dimensionnelles de fabrication des seringues, et de la présence du ressort propulseur de retour, il est quasiment impossible de garantir l'injection de la totalité de la dose renfermée dans lesdites seringues. Or, pour certains produits tels que de l'héparine ou des vaccins spécifiques, la dose de produit injectée doit être garantie de façon très précise, condition que ne peuvent remplir de façon systématique les dispositifs d'injection actuels.

De plus, la plupart des dispositifs d'injection actuels offrant cette sécurité d'utilisation pour le patient s'avèrent d'une complexité conduisant à un prix de revient difficilement compatible avec celui d'un dispositif à usage unique. En outre, de par leur conception, ils nécessitent généralement l'utilisation de seringues spécifiques dont le coût de revient est sans aucune mesure avec celui des seringues classiques utilisées sans dispositif d'injection automatique.

Ces deux derniers inconvénients ont été palliés par le dispositif d'injection décrit dans la demande de brevet WO 94/21316 qui comprend :
- un boîtier longiligne présentant une paroi frontale avant percée d'un orifice, et formé de deux parties, antérieure et postérieure emmanchées l'une dans l'autre et aptes à coulisser longitudinalement l'une par rapport à l'autre sur une faible course de façon à pouvoir subir un déplacement relatif entre deux positions : une position reculée, dite de repos, de la partie postérieure par rapport à la partie antérieure obtenue naturellement en l'absence de sollicitation externe exercée sur lesdites parties, et une position avancée, dite d'injection, de la partie postérieure par rapport à la partie antérieure obtenue par le biais d'un effort longitudinal exercé sur ladite partie postérieure,
- une seringue disposée dans le boîtier et comportant un corps de seringue logeant un piston, doté d'une bague appui-doigts, et portant une aiguille,
- une tige de piston dotée d'une collerette, et s'étendant dans le prolongement arrière de la seringue,
- des moyens de maintien de la collerette de la tige de piston,
- un ressort propulseur s'étendant entre les moyens de maintien de la collerette de la tige de piston et le fond de la partie postérieure du boîtier,
- des moyens de blocage en translation des moyens de maintien de la collerette de la tige de piston aptes à les maintenir à l'intérieur du boîtier dans une position où le ressort propulseur est comprimé, et où la seringue est entièrement logée dans ledit boîtier, et à autoriser leur déplacement vers une position avancée de fin d'injection, engendré par le ressort propulseur après déplacement des parties du boîtier de leur position reculée vers leur position avancée,
- un organe de butée du corps de seringue dans une position avancée de ce dernier où l'aiguille s'étend au moins partiellement au travers de l'orifice du boîtier,
- des moyens de déclenchement des moyens de maintien de la collerette de la tige de piston, aptes à libérer ladite collerette, dans la position avancée desdits moyens de maintien, après déplacement des parties du boîtier de leur position avancée vers leur position reculée,
- et un ressort propulseur de retour de force inférieure à celle du ressort propulseur, disposé autour du corps de seringue de façon à être comprimé par la bague appui-doigts dudit corps de seringue lors du déplacement de ce dernier vers sa position avancée.

Toutefois, un tel dispositif d'injection ne permet pas de pallier au premier inconvénient précité, à savoir de garantir l'injection de la totalité de la dose.

La présente invention vise à pallier cet inconvénient et a pour objectif de fournir un dispositif d'injection automatique conçu pour permettre d'injecter de-façon systématique une dose de produit médicamenteux strictement définie.

Un autre objectif de l'invention est de fournir un dispositif d'injection de conception simplifiée et donc d'un prix de revient compatible avec celui d'un dispositif à usage unique.

Un autre objectif de l'invention est de foumir un dispositif d'injection utilisable avec des seringues pré-remplies de type traditionnel.

A cet effet, l'invention vise un dispositif d'injection automatique d'une dose de produit médicamenteux du type décrit dans la demande de brevet WO 94/21316.

Selon l'invention, ce dispositif d'injection se caractérise en ce que :
- il comprend des moyens de visualisation ménagés dans la paroi du boîtier de façon à permettre de visualiser les moyens de maintien de la collerette de la tige de piston dans leur position avancée,
- les moyens de maintien de la collerette de la tige de piston comprennent :
   . un tube interne, dit de guidage, s'étendant dans la partie postérieure du boîtier de façon à délimiter à l'intérieur de cette dernière un espace annulaire périphérique adapté pour loger le ressort propulseur dans la position comprimée dudit ressort,
   . une cage de forme adaptée pour coulisser à l'intérieur du tube de guidage, et pour loger la tige de piston et la collerette de cette dernière, ladite cage comportant vers une de ses extrémités, dite postérieure, un organe interne de blocage de la collerette de la tige de piston apte à se déformer radialement, et vers son autre extrémité antérieure,-une paroi frontale avant de butée du ressort propulseur et de contact avec les moyens de blocage en translation desdits moyens de maintien dans la position reculée de ces derniers,
   . lesdits tube de guidage et cage étant adaptés pour que, dans la position avancée des moyens de maintien, l'organe de blocage de la collerette de la tige de piston reste emprisonné dans le tube de guidage dans la position relative avancée des parties de boîtier, et soit libéré dans la position relative reculée desdites parties de boîtier.

Selon l'invention, l'injection est réalisée en appliquant en premier lieu et de façon classique l'extrémité antérieure du boîtier contre la peau. Une pression exercée sur la partie postérieure du boîtier autorise ensuite le déclenchement du ressort propulseur et donc l'injection du produit. Au terme de cette injection, le patient possède un témoin visuel de fin de course qui l'informe que l'intégralité de la dose a été injectée. Il peut alors relâcher la pression sur la partie postérieure du boîtier, relâchement qui autorise le recul de cette partie postérieure et par la suite le retrait de la seringue dans le boîtier sous l'action du ressort propulseur de retour.

Un tel dispositif d'injection qui comprend, d'une part, un boîtier en deux parties dont le déplacement relatif permet d'absorber les tolérances de variations de longueurs des seringues et de déclencher l'injection et le retrait ultérieur de la seringue à l'intérieur du boîtier, et d'autre part; un témoin visuel de fin d'injection, permet donc de délivrer systématiquement l'intégralité du produit renfermé dans la seringue, et constitue en outre un dispositif à usage unique éliminant tout risque de piqûre avant et après usage.

De plus, l'absorption des tolérances de variation de longueurs des seringues est obtenue de façon très simple en prévoyant une marge suffisante concernant la longueur de la cage restant emprisonnée dans le tube interne, en fin d'injection.

En outre, le tube de guidage et la cage présentent préférentiellement des dimensions adaptées pour loger la tige de piston et la bague appui-doigts du corps de seringue, ladite cage comportant un organe interne de butée de la bague appui-doigts du corps de seringue dans la position reculée de ladite cage, et une paroi frontate dotée d'une ouverture apte à permettre le déplacement relatif de cette cage et du corps de seringue, dans la position avancée de ce dernier.

La présence de cet organe interne de butée de la bague appui-doigts du corps de seringue permet avantageusement d'éviter que ledit corps de seringue soit entraîné vers l'avant lors du retrait de l'embout de protection de l'aiguille. En effet, cet entraînement, constaté avec les dispositifs actuels, et qui tend à comprimer le ressort propulseur de retour, conduit à une gêne pour l'utilisateur qui constatant une résistance résultant de cette compression peut hésiter à exercer des efforts visant à vaincre cette résistance.

Selon une autre caractéristique de l'invention, ce dispositif d'injection est adapté pour être utilisé avec une seringue traditionnelle dont la bague appui-doigts présente une forme annulaire tronquée et comporte deux méplats diamétralement opposés. A cet effet, la cage se compose d'un étrier comportant deux ailes longitudinales agencées pour venir au contact chacune d'un méplat de la bague appui-doigts.

Un tel étrier présente l'avantage de constituer un élément de blocage en rotation du corps de seringue présentant un faible encombrement transversal, qui combiné à la disposition du ressort propulseur dans un espace annulaire situé autour dudit étrier, conduit à l'obtention d'un dispositif d'injection d'encombrement réduit.

Selon deux modes de réalisation préférentiels visant l'étrier :
- l'organe interne de blocage de la collerette de la tige de piston est constitué de rainures transversales ménagées en regard dans les ailes de l'étrier,
- cet étrier comprend une bague dans le prolongement arrière de laquelle s'étendent les ailes longitudinales, l'organe interne de butée de la bague appui-doigts du corps de seringue comportant au moins une languette inclinée s'étendant longitudinalement à l'intérieur de ladite bague.

Selon une autre caractéristique de l'invention :
- la partie postérieure du boîtier présente extérieurement la forme d'un fourreau de forme générale cylindrique,
- la partie antérieure du boîtier présente des dimensions adaptées pour se loger sur sa plus grande longueur à l'intérieur de la partie postérieure dudit boîtier.
- des organes de guidage sont adaptés pour permettre de déplacer sur une faible course la partie postérieure du boîtier le long de la partie antérieure dudit boîtier.

Cet agencement des parties antérieure et postérieure du boîtier permet d'obtenir un boîtier parfaitement rigide en flexion et donc d'un emploi très sécurisant pour l'utilisateur.

Selon une autre caractéristique de l'invention, les moyens de blocage en translation des moyens de maintien de la collerette de la tige de piston comprennent :
- un organe de butée déformable radialement, solidaire des moyens de maintien,
- un organe interne de butée axiale de l'organe déformable de butée, ménagé à l'intérieur de la partie postérieure du boîtier.
- une gâchette ménagée dans la paroi périphérique de la partie postérieure du boîtier de façon à pouvoir déformer et libérer l'organe de butée des moyens de maintien, dans la position reculée de ces derniers.
- et une lumière ménagée dans la paroi périphérique de la partie antérieure du boîtier, et de forme adaptée, d'une part, pour que les organes de butée s'étendent au travers de ladite lumière, et d'autre part, pour permettre l'actionnement de la gâchette uniquement dans la position relative avancée desdites parties de boîtier.

Cette disposition permet de garantir contre tout déclenchement accidentel de l'injection puisque cette dernière requiert, dans un premier temps, d'exercer une pression sur le boîtier, puis dans un deuxième temps, d'appuyer sur la gâchette de façon à escamoter l'organe de butée déformable des moyens de maintien.

Selon un mode de réalisation préférentiel, la gâchette présente une forme longitudinale en T, la lumière présentant une forme conjuguée et étant ménagée de façon à présenter une branche transversale décalée longitudinalement de la barre transversale de la gâchette dans la position relative reculée des parties du boîtier.

Selon une autre caractéristique de l'invention, le dispositif d'injection comprend un capuchon de forme adaptée pour coiffer le tronçon d'extrémité avant de la partie antérieure du boîtier et venir en butée contre la partie postérieure dudit boîtier dans la position reculée de cette dernière.

Un tel capuchon, outre sa fonction classique de protection, permet de garantir le blocage relatif en translation, avant usage, des parties antérieure et postérieure du boîtier dans la position reculée desdites parties.

Par ailleurs, la partie antérieure du boîtier intègre avantageusement un conduit interne de guidage du corps de seringue s'étendant sur une portion de la longueur de ladite partie antérieure à partir de la paroi frontale de cette dernière, ledit conduit présentant un épaulement de butée dudit corps de seringue dans sa position avancée.

Le capuchon comporte par ailleurs préférentiellement des griffes s'étendant longitudinalement à l'intérieur dudit capuchon et disposées de façon à se loger dans le conduit interne de guidage du corps de seringue, lesdites griffes étant adaptées pour venir se crocheter sur un embout de protection de l'aiguille de la seringue.

Cette disposition permet de retirer automatiquement l'embout protégeant de façon classique l'aiguille, lors du retrait du capuchon.

De plus, selon une autre caractéristique de l'invention, la partie postérieure du boîtier et le capuchon présentent des faces de contact profilées en forme de came aptes à permettre le retrait dudit capuchon par un mouvement de rotation de ce dernier.

D'autres caractéristiques, buts et avantages de l'invention ressortiront de la description détaillée qui suit en référence aux dessins annexés qui en représentent à titre d'exemple non limitatif un mode de réalisation préférentiel. Sur ces dessins qui font partie intégrante de la présente description :

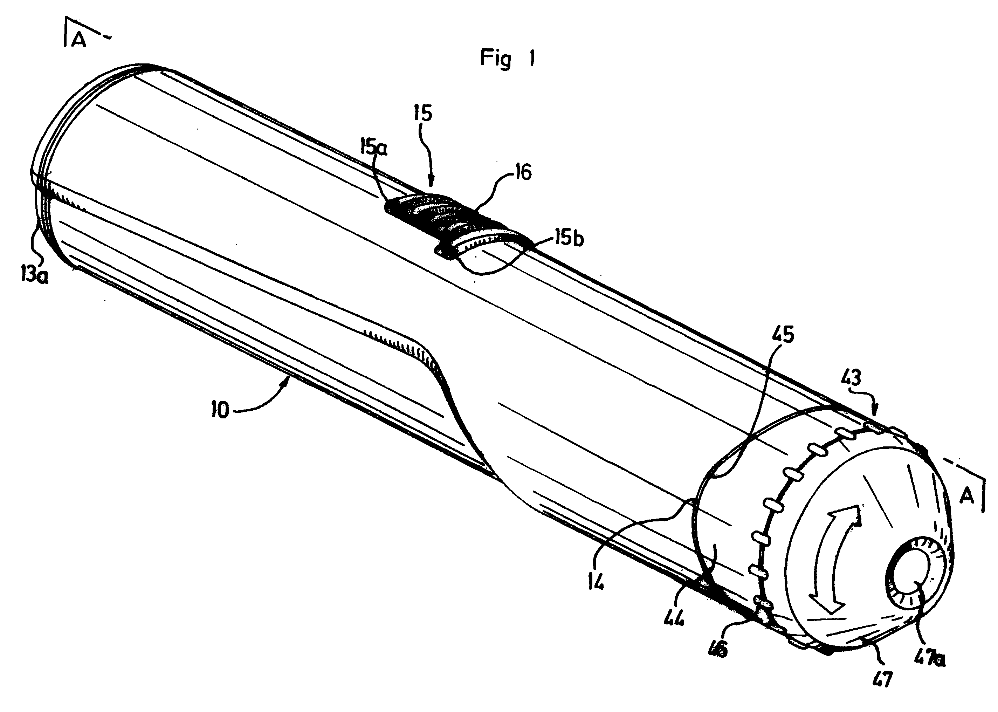

- la figure 1 est une vue en perspective d'un dispositif d'auto-injection conforme à l'invention,
- la figure 2 est une coupe longitudinale par un plan axial A de la partie postérieure du boîtier de ce dispositif d'auto-injection,
- la figure 3 est une coupe longitudinale par un plan axial B de cette partie postérieure de boîtier,
- la figure 4 est une coupe longitudinale par le plan axial A de la partie antérieure du boîtier de ce dispositif d'auto-injection,
- la figure 5 est une vue longitudinale de dessus de cette partie antérieure de boîtier,
- la figure 6 est une vue en perspective de l'étrier de maintien de la tige du piston de seringue,
- la figure 7 est une coupe longitudinale par le plan axial A du capuchon de ce dispositif d'auto-injection,
- les figures 8 à 13 sont des coupes longitudinales par le plan axial A du dispositif d'auto-injection conforme à l'invention, illustrant les étapes successives du fonctionnement de ce dispositif,
- les figures 14 et 15 sont des vues en perspective schématiques partielles représentant le dispositif d'auto-injection respectivement dans la position reculée et la position avancée du tronçon postérieur, le tronçon antérieur dudit dispositif d'auto-injection étant matérialisé en pointillé,
- et la figure 16 est une coupe transversale par un plan C de ce dispositif d'auto-injection.

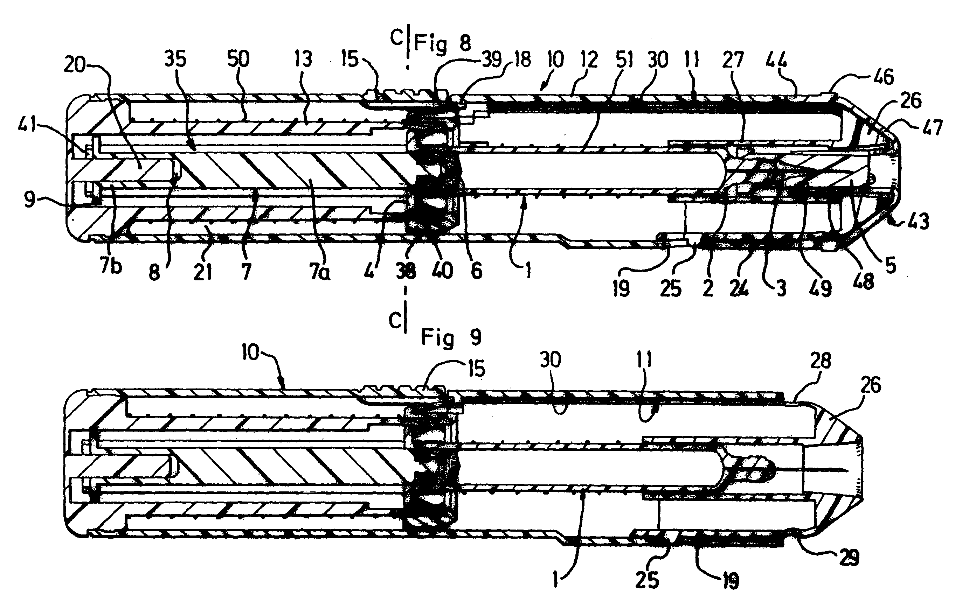

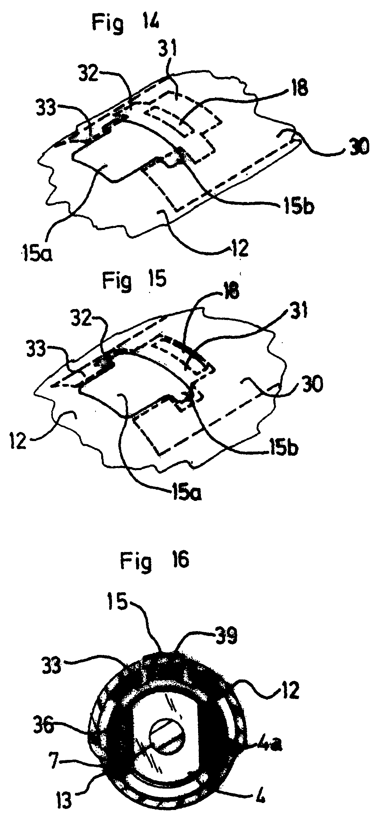

Le dispositif d'auto-injection conforme à l'invention, représenté aux figures 8 à 13, est du type à usage unique, et est adapté pour permettre à un patient de s'auto-administrer une dose de liquide médicamenteux renfermé dans une seringue pré-remplie 1 de type traditionnel, telle que par exemple réalisée en verre, et comportant de façon classique :
- un col 2 sur lequel est monté une aiguille d'injection 3, une bague appui-doigts 4 postérieure présentant une forme annulaire tronquée, c'est-à-dire possédant, tel que représenté à la figure 16, deux méplats tels que 4a diamétralement opposés,
- un embout 5 de protection de l'aiguille d'injection 3 dans le fond duquel l'extrémité de ladite aiguille est plantée afin d'éviter tout écoulement de liquide,
- un bouchon 6 faisant office de piston,
- et une tige de piston 7 formée sur sa plus grande longueur d'une tige 7a de section cruciforme prolongée d'un tronçon postérieur tubulaire 7b percé d'un alésage borgne axial 8 débouchant au niveau d'une collerette postérieure 9 de forme générale carrée.

Ce dispositif d'auto-injection comporte, en premier lieu, un boîtier de forme externe générale cylindrique composé de deux parties dites postérieure 10 et antérieure 11 emmanchées l'une dans l'autre de façon à pouvoir coulisser longitudinalement l'une à l'intérieur de l'autre sur une faible course de l'ordre de quelques millimètres.

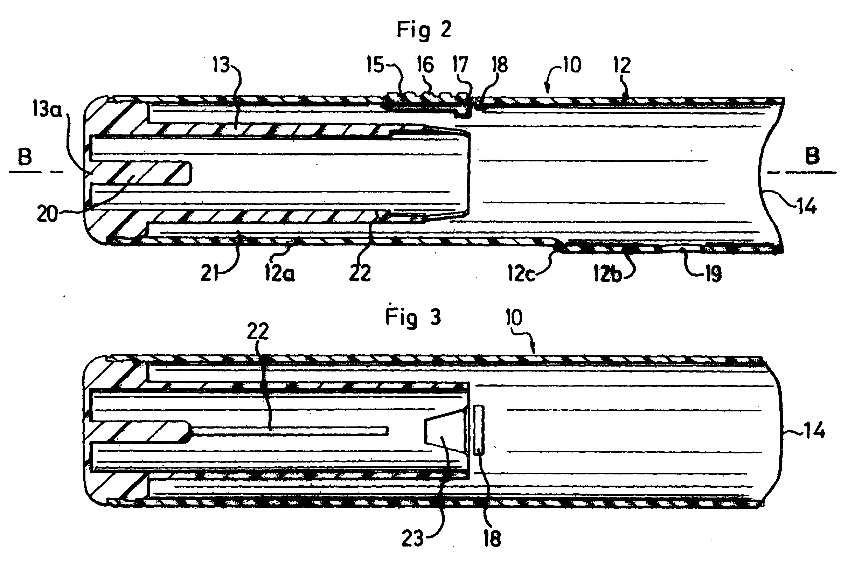

Tel que représenté aux figures 2 et 3, la partie postérieure 10 du boîtier est constituée, à des fins de réalisation par injection d'une matière plastique, de deux éléments 12, 13 aptes à être assemblés après fabrication de façon à former un ensemble d'un seul tenant.

Le premier de ces éléments 12 présente la forme d'un fourreau de forme générale cylindrique doté d'une extrémité antérieure 14 profilée en forme de came.

Ce fourreau 12 est, en outre, doté d'une "gâchette" 15 délimitée par une fente 17 ménagée dans sa paroi périphérique, et adaptée pour pivoter longitudinalement et s'escamoter partiellement à l'intérieur dudit fourreau sous l'effet d'une pression manuelle.

Cette gâchette 15 présente longitudinalement la forme d'un T dont la barre longitudinale 15a est orientée en direction de l'extrémité postérieure du fourreau 12 et présente, vu en plan, une forme rectangulaire adaptée pour être pressée par un doigt, de façon que la barre transversale 15b s'escamote à l'intérieur du fourreau 12 lors d'une telle pression.

Cette gâchette 15 présente, enfin, une face supérieure striée dotée de nervures transversales telles que 16 faisant saillie par rapport à la paroi périphérique du fourreau 12.

Le fourreau 12 comporte, par ailleurs, une nervure interne transversale 18 en forme de secteur circulaire, ménagée en regard de la barre transversale 15b de la gâchette 15 et séparée de cette dernière par la portion transversale de la fente 17.

Ce fourreau 12 comporte, enfin, une lumière oblongue 19 à grand axe longitudinal, ménagée dans le quart de tronçon antérieur dudit fourreau, au niveau d'une génératrice diamétralement opposée à la gâchette 15.

Le deuxième élément 13 du tronçon postérieur 10 consiste en un tube 13 de forme générale cylindrique, obturé au niveau de son extrémité postérieure par un embout 13a de forme adaptée pour venir s'emmancher dans le premier élément 12. Cet embout 13a comporte, en outre, un pion de centrage 20 en saillie par rapport à sa paroi frontale, de façon à s'étendre axialement dans le fourreau 12, sur une portion de longueur de ce dernier.

Ce tube 13 présente un diamètre externe inférieur au diamètre interne du premier élément 12 adapté pour délimiter un volume annulaire 21 à l'intérieur de ce dernier.

Le tube 13 comporte, par ailleurs, des nervures internes longitudinales telles que 22 réparties autour de l'axe de ce dernier, s'étendant à partir de l'embout 13a et interrompues à distance de l'extrémité antérieure dudit tube, de façon à former un épaulement de butée axiale à l'intérieur de ce dernier.

Ce tube 13 comporte, enfin, deux échancrures telles que 23 diamétralement opposées, présentant vue en plan, une forme trapézoïdale.

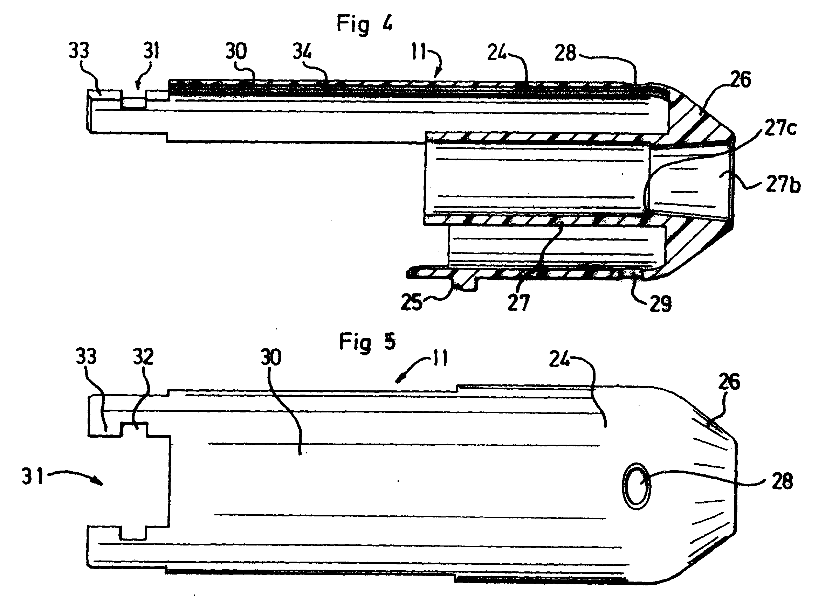

Tel que représenté aux figures 4 et 5, la partie antérieure 11 du boîtier comporte un étui cylindrique 24 de diamètre externe adapté pour venir se loger à l'intérieur de la partie postérieure 10 dudit boîtier. Au niveau de son tronçon d'extrémité postérieure, cet étui 24 est doté d'un pion externe 25 de forme adaptée pour venir se loger dans la lumière oblongue 19 de la partie postérieure 10. Ce pion externe 25 présente, en outre, un diamètre inférieur à la longueur de la lumière oblongue 19, adapté pour permettre à la partie antérieure 11 de coulisser longitudinalement sur une faible course de l'ordre de quelques millimètres à l'intérieur de la partie postérieure 10 du boîtier.

Au niveau de son extrémité antérieure, l'étui 24 est prolongé par un nez tubulaire 26 de forme générale tronconique, percé axialement d'un alésage 27b tronconique de section décroissante à partir de la face d'extrémité dudit nez tubulaire.

Cet alésage tronconique 27b est, en outre, ménagé dans le prolongement d'un conduit cylindrique 27 de guidage du corps de seringue, s'étendant axialement à l'intérieur de l'étui cylindrique 10, et séparé dudit alésage tronconique par un épaulement 27c de butée axiale dudit corps de seringue.

L'étui cylindrique 24 est, par ailleurs, percé à proximité du nez tubulaire 26, de deux lumières radiales 28, 29 diamétralement opposées.

La partie antérieure 11 du boîtier comporte, en outre, une aile longitudinale postérieure 30 s'étendant dans le prolongement de l'étui cylindrique 24, et constituée d'une portion de la paroi périphérique dudit étui cylindrique délimitant un secteur circulaire d'angle au sommet de l'ordre de 60°.

Cette aile longitudinale 30 comprend, en premier lieu, une lumière axiale 31 ménagée à partir de son extrémité postérieure, de largeur supérieure à celle de la barre longitudinale 15a de la gâchette 15 et inférieure à la longueur de la barre transversale 15b de ladite gâchette.

Cette aile longitudinale 30 comprend, en outre, deux échancrures telles que 32 ménagées en vis-à-vis, de façon à déboucher dans la lumière 31 sensiblement à mi-longueur de cette dernière et à délimiter une fente transversale de longueur supérieure à celle de la barre transversale 15b de la gâchette 15, séparée de l'extrémité postérieure de ladite aile longitudinale par une plage de paroi en forme de créneau 33.

Cette aile longitudinale 30 comprend, enfin, une rainure longitudinale 34 ménagée axialement dans le prolongement de la lumière 31.

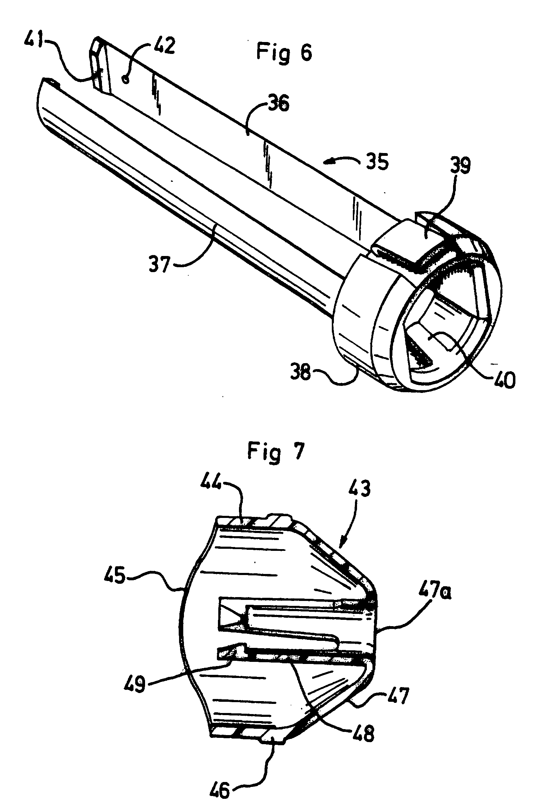

Le dispositif d'auto-injection selon l'invention comporte, en outre, un étrier 35 représenté à la figure 6 de maintien et d'entraînement de la tige de piston 7 de la seringue 1.

Cet étrier 35 présente en premier lieu deux ailes longitudinales parallèles 36, 37 espacées d'une distance et d'une section adaptées pour pouvoir coulisser dans le tube 13 de la partie postérieure 10.

Cet étrier 35 comprend, en outre, une bague annulaire 38 d'un seul tenant avec les ailes longitudinales 36, 37 et à l'intérieur de laquelle sont ménagés les tronçons d'extrémités antérieurs desdites ailes.

Côté externe, cette bague annulaire 38 présente une échancrure à l'intérieur de laquelle s'étend une languette déformable 39 solidarisée à ladite bague au niveau de son extrémité postérieure.

Côté interne, cette bague 39 comporte deux languettes inclinées telles que 40, décalées de 90° par rapport aux ailes longitudinales 36, 37, et orientées en direction de l'extrémité postérieure de l'étrier 35.

Chaque aile longitudinale 36, 37 de l'étrier 35 comporte, par ailleurs, une nervure transversale interne telle que 41 ménagée au niveau de l'extrémité postérieure de ladite aile, et à faible distance de ladite nervure, un bouton axial 42 en saillie par rapport à la face interne de cette aile 36, 37.

Le dispositif d'auto-injection comprend également un capuchon 43 adapté pour venir coiffer le tronçon d'extrémité de la partie antérieure 11 du boîtier, et comportant à cet effet, un tronçon cylindrique 44 prolongé d'un nez tronconique 47, de dimensions conjuguées de celles de l'étui cylindrique 24 et du nez tubulaire 26.

L'extrémité postérieure 45 du tronçon cylindrique 44 de ce capuchon 43 présente, en outre, la forme d'une came conjuguée de celle du fourreau 12 de la partie postérieure 10 du boîtier, adaptée pour permettre le retrait dudit capuchon par un mouvement de rotation imprimé à ce dernier dans l'un ou l'autre sens de rotation.

Le capuchon 43 comporte également des stries externes telles que 46 ménagées sur le tronçon cylindrique 44 et formant un moletage de préhension manuelle dudit capuchon.

Le capuchon 43 comporte, enfin, une ouverture frontale axiale 47a ménagée dans le nez tronconique 47 et, s'étendant dans ce capuchon 43 à partir de cette ouverture frontale 47a, trois griffes longitudinales 48 uniformément autour de l'axe dudit capuchon.

Ces griffes 48 dotées d'extrémités en forme de crochets 49 sont ménagées de façon à être resserrées lors de la mise en place du capuchon 43 sur la partie antérieure 11 du boîtier, du fait de la forme tronconique de l'alésage 27b de ladite partie antérieure, et à venir se crocheter sur l'embout 45 de protection de l'aiguille 3 de la seringue 1 en vue de permettre le retrait de ce dernier.

Le dispositif d'auto-injection comprend enfin deux ressorts propulseurs 50, 51 :
- un ressort propulseur d'injection 50 logé dans l'espace annulaire 21 de la partie postérieure 10 du boîtier de façon à s'étendre entre la paroi frontale de l'embout 13a et la bague 38 de l'étrier 35,
- un ressort propulseur de retour 51 de force inférieure à celle du ressort propulseur 50, disposé autour de la seringue 1 de façon à s'étendre entre l'extrémité postérieure du conduit cylindrique 27 et la bague appui-doigts 4 de ladite seringue.

Le fonctionnement du dispositif d'auto-injection et les caractéristiques dimensionnelles des différents éléments constitutifs autorisant ce fonctionnement sont décrits ci-dessous en référence aux figures 8 à 13.

En premier lieu, tel que représenté à la figure 8, l'assemblage initial des divers éléments conduit à :
- positionner la seringue 1 relativement à l'étrier 35 de façon que la bague appui-doigts 4 soit disposée en butée contre les languettes 40 dudit étrier, et la collerette 9 de la tige du piston 7 positionnée entre les nervures 41 et les boutons 42. De plus, le pion de centrage 20 s'étend alors dans l'alésage borgne 8 de la tige de piston 7,
- positionner l'étrier 35 à l'intérieur du tube interne 13 de façon que la languette 39 dudit étrier vienne en butée axiale contre la nervure interne 18 du fourreau 12, tandis que les languettes 40 viennent se loger dans les échancrures 23 dudit tube interne,

Tel que cela ressort de la figure 8 et selon ces dispositions :
- le ressort propulseur d'injection 50 est maintenu comprimé entre la paroi frontale 13a du tube 13 et la bague 38 de l'étrier 35,
- la seringue 1 est entièrement logée dans le boîtier, l'extrémité de l'embout de protection 5 se situant en retrait de l'extrémité du nez 26,
- l'extrémité postérieure du capuchon 43 et l'extrémité antérieure de la partie postérieure 10 du boîtier sont au contact l'une de l'autre, de sorte que, du fait que le capuchon 43 est solidaire de la partie antérieure 11 du boîtier, tout déplacement relatif des parties antérieure 11 et postérieure 10 du boîtier est interdit,
- la partie postérieure 10 du boîtier se trouve dans une position « reculée » par rapport à la partie antérieure 11 dudit boîtier, position dans laquelle, tel que représenté à la figure 14, la barre transversale 15b de la gâchette 15 se trouve positionnée en regard des créneaux 33, interdisant d'actionner ladite gâchette.

En vue d'une injection, la première opération consiste à retirer le capuchon 43 et simultanément l'embout 5 protège aiguille 3. Ce retrait est obtenu en faisant tourner le capuchon 43 dans l'un ou l'autre sens, rotation au cours de laquelle les profils en forme de came 14, 45 conduisent à entraîner un déplacement axial dudit capuchon, de sorte que ledit retrait s'assimile à une simple opération de dévissage.

En outre, lors de ce retrait, la seringue 1 ne subit aucune translation du fait que la bague appui-doigts 4 est en butée contre les languettes 40 de l'étrier 35, de sorte qu'aucune compression du ressort propulseur de retour 51 n'intervient.

L'opération suivante consiste à appliquer le nez 26 du boîtier contre la peau et à exercer une pression longitudinale sur la partie postérieure 10 dudit boîtier. Tel que représenté à la figure 9, cette opération amène la partie postérieure 10 du boîtier à se déplacer vers l'avant relativement à la partie antérieure 11, déplacement au cours duquel le guidage relatif des dites parties est assuré par le pion 25 logé dans la lumière 19.

Au terme de ce déplacement relatif, et tel que représenté sur les figures 9 et 15, la barre transversale 15b de la gâchette 15 se trouve positionnée en regard des échancrures 32, de sorte que ladite gâchette peut être actionnée.

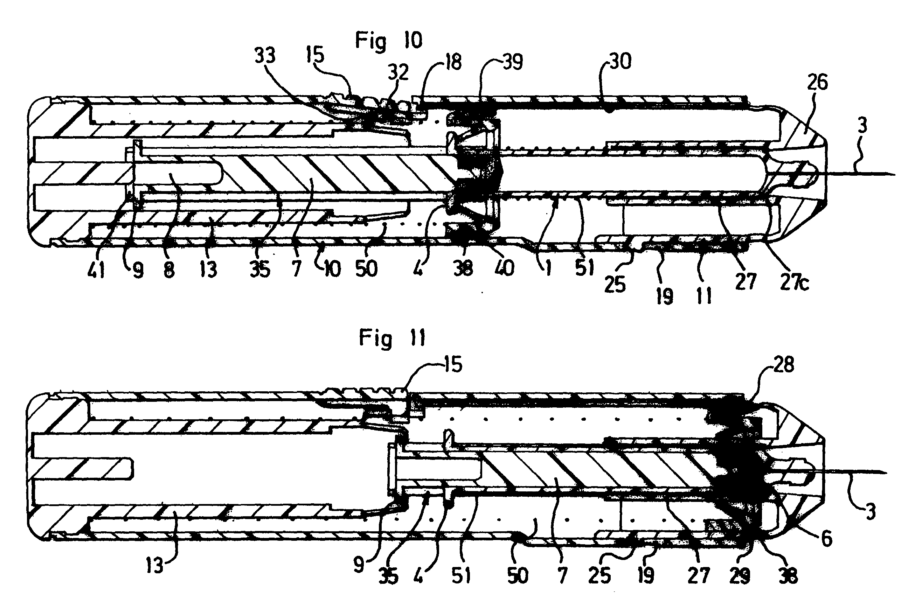

Tel que représenté à la figure 10, l'injection est ensuite obtenue par un actionnement de la gâchette 15 qui conduit à déformer la languette 39 et à libérer l'étrier 35, qui sous l'effet du ressort propulseur 50 :
- déplace dans un premier temps l'ensemble seringue 1/tige de piston 7 jusqu'à ce que le corps de seringue vienne en butée contre l'épaulement 27c du conduit 27, déplacement au cours duquel l'aiguille 3 vient se planter dans le corps du patient (figure 10). (De plus, au cours de ce déplacement, la languette 39 se déplace dans la rainure 34, de sorte que les forces de frottement de cette dernière sont minimisées).
- déplace ensuite la tige de piston 7 relativement au corps de seringue 1 conduisant à l'injection du produit renfermé dans ladite seringue.

Au terme de cette injection et tel que représenté à la figure 11, la bague 38 de l'étrier 35 vient se positionner en regard des lumières 28, 29 du boîtier de sorte que le patient peut visualiser cette bague 38 et ainsi être assuré de la complète vidange de la seringue 1.

Cette vidange complète est en outre assurée tel que représenté à la figure 11, du fait que l'extrémité postérieure des ailes 36, 37 de l'étrier 35 reste toujours positionnée à l'intérieur du tube interne 13 en fin de course dudit étrier, de sorte que la collerette 9 de la tige de piston 7 est maintenue pincée entre lesdites ailes.

Une fois la fin d'injection constatée par le patient, ce dernier n'a plus qu'à relâcher la pression exercée sur le boîtier. Cette action a pour effet de conduire à un recul de la partie postérieure 10 par rapport à la partie antérieure 11, sous l'effet de la force rémanente du ressort propulseur d'injection 50.

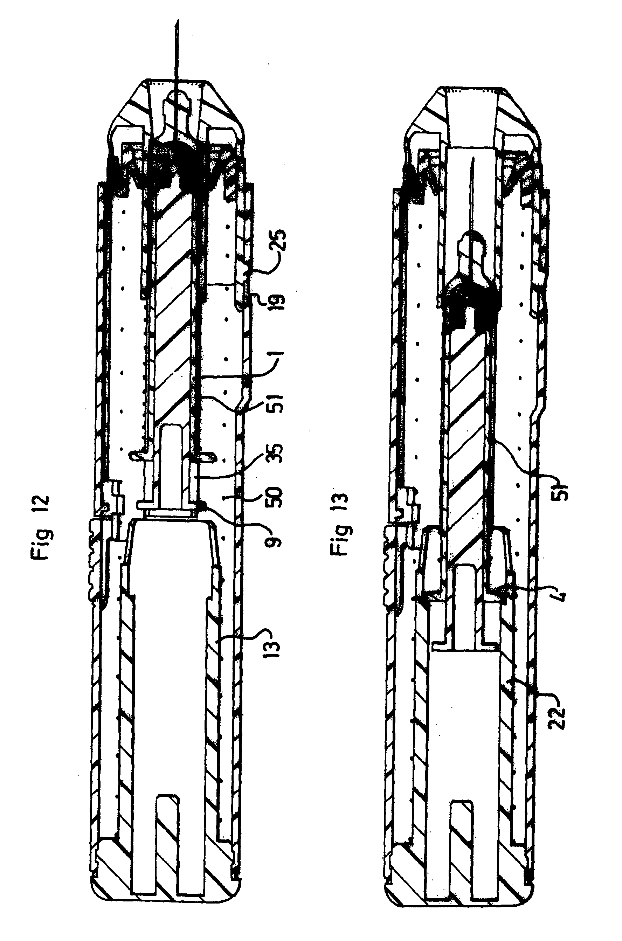

Tel que représenté à la figure 12, ce recul conduit à libérer l'extrémité postérieure des ailes 36, 37 de l'étrier 35 qui n'assurent donc plus leur fonction de maintien de la collerette 9 de la tige de piston 7.

Tel que représenté à la figure 13, la seringue 1 se trouve alors propulsée à l'intérieur du boîtier sous l'action du ressort propulseur de retour 51 jusqu'à amener la bague appui-doigts 4 en butée contre l'extrémité des nervures 22.

Le dispositif d'injection peut alors être jeté sans aucun risque de piqûre ultérieure du fait, d'une part que l'aiguille 3 se trouve nettement en retrait de l'extrémité du nez 26 du boîtier, et d'autre part que ledit dispositif ne peut pas être réactivé.

## Revendications

1. Dispositif d'injection automatique d'une dose de produit médicamenteux comprenant :
- un boîtier longiligne présentant une paroi frontale avant (26) percée d'un orifice (27b), et formé de deux parties, antérieure (11) et postérieure (10) emmanchées l'une dans l'autre et aptes à coulisser longitudinalement l'une par rapport à l'autre sur une faible course de façon à pouvoir subir un déplacement relatif entre deux positions : une position reculée, dite de repos, de la partie postérieure (10) par rapport à la partie antérieure (11) obtenue naturellement en l'absence de sollicitation externe exercée sur lesdites parties, et une position avancée, dite d'injection, de la partie postérieure (10) par rapport à la partie antérieure (11) obtenue par le biais d'un effort longitudinal exercé sur ladite partie postérieure,
- une seringue (1) disposée dans le boîtier et comportant un corps de seringue logeant un piston (6), doté d'une bague appui-doigts (4), et portant une aiguille (3),
- une tige de piston (7) dotée d'une collerette (9), et s'étendant dans le prolongement arrière de laseringue (1),
- des moyens de maintien de la collerette (9) de la tige de piston (7),
- un ressort propulseur (50) s'étendant entre les moyens de maintien (35) de la collerette (9) de la tige de piston (7) et le fond de la partie postérieure (10) du boîtier,
- des moyens (15, 18, 39) de blocage en translation des moyens de maintien (35) de la collerette (9) de la tige de piston (7) aptes à les maintenir à l'intérieur du boîtier dans une position où le ressort propulseur (50) est comprimé, et où la seringue (1) est entièrement logée dans ledit boîtier, et à autoriser leur déplacement vers une position avancée de fin d'injection, engendré par le ressort propulseur (50) après déplacement des parties (10, 11) du boîtier de leur position reculée vers leur position avancée,
- un organe (27c) de butée du corps de seringue (1) dans une position avancée de ce dernier où l'aiguille (3) s'étend au moins partiellement au travers de l'orifice (27b) du boîtier,
- des moyens (13) de déclenchement des moyens de maintien (35) de la collerette (9) de la tige de piston (7), aptes à libérer ladite collerette, dans la position avancée desdits moyens de maintien, après déplacement des parties (10, 11) du boîtier de leur position avancée vers leur position reculée,
- et un ressort, propulseur de retour (51) de force inférieure à celle du ressort propulseur (50), disposé autour du corps de seringue (1) de façon à être comprimé par la bague appui-doigts (4) dudit corps de seringue lors du déplacement de ce dernier vers sa position avancée,
ledit dispositif d'injection étant **caractérisé en ce que** :
- il comprend des moyens de visualisation (28, 29) ménagés dans la paroi du boîtier de façon à permettre de visualiser les moyens de maintien (35) de la collerette (9) de la tige de piston (7) dans leur position avancée,
- les moyens de maintien de la collerette (9) de la tige de piston (7) comprennent :
. un tube interne (13), dit de guidage, s'étendant dans la partie postérieure (10) du boîtier de façon à délimiter à l'intérieur de cette dernière un espace annulaire périphérique (21) adapté pour loger, le ressort propulseur (50) dans la position comprimée dudit ressort,
. une cage (35) de forme adaptée pour coulisser à l'intérieur du tube de guidage (13), et pour loger la tige de piston (7) et la collerette (9) de cette dernière, ladite cage comportant vers une de ses extrémités, dite postérieure, un organe inteme (41) de blocage de la cotferette (9) de la tige de piston (7) apte à se déformer radialement, et vers son autre extrémité antérieure, une paroi frontale avant (38) de butée du ressort propulseur (50) et de contact avec les moyens de blocage en translation (15, 18, 39) desdits moyens de maintien dans la position reculée de ces derniers,
. lesdits tube de guidage et cage étant adaptés pour que, dans la position avancée des moyens de maintien, l'organe de blocage (41) de la collerette (9) de la tige de piston (7) reste emprisonné dans le tube de guidage (13) dans la position relative avancée des parties (10, 11) de boîtier, et soit libéré dans la position relative reculée desdites parties de boîtier.

2. Dispositif d'injection selon la revendication 1, **caractérisé en ce que** le tube de guidage (13) et la cage (35) présentent des dimensions adaptées pour loger la tige de piston (7) et la bague appui-doigts (4) du corps de seringue (1), ladite cage comportant un organe interne (40) de butée de la bague appui-doigts (4) du corps de seringue (1) dans la position reculée de ladite cage, et une paroi frontale (38) dotée d'une ouverture apte à permettre le déplacement relatif de cette cage (35) et du corps de seringue (1), dans la position avancée de ce dernier.

3. Dispositif d'injection selon la revendication 2, dans lequel la bague appui-doigts (4) du corps de seringue (1) présente une forme annulaire tronquée et comporte deux méplats (4a) diamétralement opposés, **caractérisé en ce que** la cage (35) comporte un étrier doté de deux ailes longitudinales (36, 37) agencées pour venir au contact chacune d'un méplat (4a) de la bague appui-doigts (4).

4. Dispositif d'injection selon la revendication 3, **caractérisé en ce que** l'organe interne de blocage de la collerette (9) de la tige de piston (7) est constitué de rainures transversales (41) ménagées en regard dans les ailes (36, 37) de l'étrier (35).

5. Dispositif d'injection selon l'une des revendications 3 ou 4, **caractérisé en ce que** l'étrier (35) comprend une bague (38) dans le prolongement arrière de laquelle s'étendent les ailes longitudinales (36, 37), l'organe interne de butée de la bague appui-doigts (4) du corps de seringue (1) comportant au moins une languette inclinée (40) s'étendant longitudinalement à l'intérieur de ladite bague.

6. Dispositif d'injection selon l'une des revendications précédentes, **caractérisé en ce que** :
- la partie postérieure (10) du boîtier présente extérieurement la forme d'un fourreau de forme générale cylindrique,
- la partie antérieure (11) du boîtier présente des dimensions adaptées pour se loger sur sa plus grande longueur à l'intérieur de la partie postérieure (10) dudit boîtier.
- des organes de guidage (19, 25) sont adaptés pour permettre de déplacer sur une faible course la partie postérieure (10) du boîtier le long de la partie antérieure (11) dudit boîtier.

7. Dispositif d'injection selon la revendication 6, **caractérisé en ce que** les moyens de blocage en translation des moyens de maintien (35) de la collerette (9) de la tige de piston (7) comprennent :
- un organe de butée (39) déformable radialement, solidaire des moyens de maintien (35),
- un organe interne (18) de butée axiale de l'organe déformable de butée (39), ménagé à l'intérieur de la partie postérieure (10) du boîtier.
- une gâchette (15) ménagée dans la paroi périphérique de la partie postérieure (10) du boîtier de façon à pouvoir déformer et libérer l'organe de butée (39) des moyens de maintien (35), dans la position reculée de ces derniers.
- et une lumière (31) ménagée dans la paroi périphérique de la partie antérieure (11) du boîtier, et de-forme adaptée, d'une part, pour que les organes de butée (18, 39) s'étendent au travers de ladite lumière, et d'autre part, pour permettre l'actionnement de la gâchette (15) uniquement dans la position relative avancée desdites parties de boîtier.

8. Dispositif d'injection selon la revendication 7, **caractérisé en ce que** la gâchette (15) présente une forme longitudinale en T, la lumière (31) présentant une forme conjuguée et étant ménagée de façon à présenter une branche transversale (32) décalée longitudinalement de la barre transversale (15b) de la gâchette (15) dans la position relative reculée des parties (10, 11) du boîtier.

9. Dispositif d'injection selon l'une des revendications 6 à 8, **caractérisé en ce qu'**il comprend un capuchon (43) de forme adaptée pour coiffer le tronçon d'extrémité avant de la partie antérieure (11) du boîtier et venir en butée contre la partie postérieure (10) dudit boîtier dans la position reculée de cette dernière.

10. Dispositif d'injection selon la revendication 9, **caractérisé en ce que** la partie postérieure (10) du boîtier et le capuchon (43) présentent des faces de contact (14, 45) profilées en forme de came aptes à permettre le retrait dudit capuchon par un mouvement de rotation de ce dernier.

11. Dispositif d'injection selon l'une des revendications précédentes, **caractérisé en ce que** la partie antérieure (11) du boîtier intègre un conduit interne (27) de guidage du corps de seringue (1) s'étendant sur une portion de la longueur de ladite partie antérieure à partir de la paroi frontale (26) de cette dernière, ledit conduit présentant un épaulement (27c) de butée dudit corps de seringue dans sa position avancée.

12. Dispositif d'injection selon la revendication 11, **caractérisé en ce que** le capuchon (35) comporte des griffes (48) s'étendant longitudinalement à l'intérieur dudit capuchon et disposées de façon à se loger dans le conduit interne (27) de guidage du corps de seringue (1), lesdites griffes étant adaptées pour venir se crocheter sur un embout (5) de protection de l'aiguille (3) de la seringue (1).

## Patentansprüche

1. Vorrichtung zum automatischen Injizieren einer Dosis eines Medikamentenprodukts, umfassend:
- ein längliches Gehäuse mit einer von einer Öffnung (27b) durchbohrten vorderen Frontwand (26) und aus zwei Teilen, einem vorderen (11) und einem hinteren (10), gebildet, die ineinander gesteckt und so gestaltet sind, dass sie in Längsrichtung in Bezug aufeinander über eine geringe Strecke gleiten können, so dass sie zwischen zwei Positionen relativ verschoben werden können: einer eingefahrenen Position, Ruheposition genannt, des hinteren Teils (10) in Bezug auf den vorderen Teil (11), die auf natürliche Weise in Abwesenheit einer externen Beanspruchung erzielt wird, die auf die genannten Teile aufgebracht wird, und einer ausgefahrenen Position, Injektionsposition genannt, des hinteren Teils (10) in Bezug auf den vorderen Teil (11), die durch die Längsvorspannkraft erzielt wird, die auf den genannten hinteren Teil wirkt,
- eine in dem Gehäuse angeordnete Spritze (1), die einen Spritzenkörper umfasst, der einen Kolben (6) aufnimmt, mit einem Fingerstützring (4) versehen ist und eine Nadel (3) trägt,
- eine Kolbenstange (7), die mit einem Kragen (9) versehen ist und in der Verlängerung hinter der Spritze (1) verläuft,
- Mittel zum Halten des Kragens (9) der Kolbenstange (7),
- eine Vortriebsfeder (50), die zwischen den Haltemitteln (35) des Kragens (9) der Kolbenstange (7) und dem Boden des hinteren Teils (10) des Gehäuses verläuft,
- Mittel (15, 18, 39) zum translationalen Arretieren der Haltemittel (35) des Kragens (9) der Kolbenstange (7), die so gestaltet sind, dass sie sie im Inneren des Gehäuses in einer Position halten, in der die Vortriebsfeder (50) komprimiert ist, und in der die Spritze (1) vollkommen in dem genannten Gehäuse aufgenommen wird, und um ihre Verschiebung in eine ausgefahrene Position des Injektionsendes zuzulassen, erzeugt durch die Vortriebsfeder (50) nach der Verschiebung der Teile (10, 11) des Gehäuses von ihrer eingefahrenen Position in Richtung auf ihre ausgefahrene Position,
- einen Anschlagmechanismus (27c) für den Spritzenkörper (1) in einer ausgefahrenen Position der Letzteren, in der die Nadel (3) wenigstens teilweise durch die Öffnung (27b) des Gehäuses verläuft,
- Mittel (13) zum Auslösen von Haltemitteln (35) des Kragens (9) der Kolbenstange (7), die so gestaltet sind, dass sie den genannten Kragen in der ausgefahrenen Position der genannten Haltemittel nach der Verschiebung der Teile (10, 11) des Gehäuses von ihrer ausgefahrenen Position in ihre eingefahrene Position freigeben,
- und eine Rückholvortriebsfeder (51) mit einer geringeren Kraft als der der Vortriebsfeder (50), die so um den Spritzenkörper (1) angeordnet ist, dass sie von dem Fingerstützring (4) des genannten Körpers der Spritze während der Verschiebung der Letzteren in ihre ausgefahrene Position komprimiert wird,
wobei die genannte Injektionsvorrichtung **dadurch gekennzeichnet ist, dass**:
- sie Sichtmittel (28, 29) umfasst, die in der Wand des Gehäuses so angeordnet sind, dass die Haltemittel (35) des Kragens (9) der Kolbenstange (7) in ihrer ausgefahrenen Position sichtbar sind,
- die Haltemittel des Kragens (9) der Kolbenstange (7) Folgendes umfassen:
. eine Innenröhre (13), Führungsröhre genannt, die im hinteren Teil (10) des Gehäuses so verläuft, dass sie im Inneren des Letzteren einen ringförmigen Umfangsraum (21) begrenzt, der die Aufgabe hat, die Vortriebsfeder (50) in der komprimierten Position der genannten Feder aufzunehmen,
. einen Käfig (35) mit einer Form, die geeignet ist, im Inneren der Führungsröhre (13) zu gleiten und die Kolbenstange (7) und den Kragen (9) der Letzteren aufzunehmen, wobei der genannte Käfig in Richtung auf eines seiner Enden, hinteres Ende genannt, einen inneren Arretierungsmechanismus (41) des Kragens (9) der Kolbenstange (7) umfasst, der sich radial verformen kann, und in Richtung auf sein anderes vorderes Ende eine vordere Frontwand (38) als Widerlager für die Vortriebsfeder (50) und für den Kontakt mit translationalen Arretierungsmitteln (15, 18, 39) für die.genannten Haltemittel in der eingefahrenen Position der Letzteren umfasst,
. wobei die genannte Führungsröhre und der Käfig so gestaltet sind, dass in der ausgefahrenen Position der Haltemittel der Arretierungsmechanismus (41) des Kragens (9) der Kolbenstange (7) in der Führungsröhre (13) in der relativen ausgefahrenen Position der Teile (10, 11) des Gehäuses gefangen bleibt und in der relativen eingefahrenen Position der genannten Teile des Gehäuses befreit wird.

2. Injektionsvorrichtung nach Anspruch 1, **dadurch gekennzeichnet, dass** die Führungsröhre (13) und der Käfig (35) Abmessungen aufweisen, die geeignet sind, die Kolbenstange (7) und den Fingerabstützring (4) des Spritzenkörpers (1) aufzunehmen, wobei der genannte Käfig einen inneren Widerlagermechanismus (40) des Fingerabstützrings (4) des Spritzenkörpers (1) in der eingefahrenen Position des genannten Käfigs und eine Frontwand (38) umfasst, die mit einer Öffnung versehen ist, die die relative Verschiebung dieses Käfigs (35) und des Spritzenkörpers (1) in der ausgefahrenen Position der Letzteren zulässt.

3. Injektionsvorrichtung nach Anspruch 2, in der der Fingerabstützring (4) des Spritzenkörpers (1) eine kegelstumpfförmige Ringform aufweist und zwei diametral gegenüberliegende Abflachungen (4a) umfasst, **dadurch gekennzeichnet, dass** der Käfig (35) einen Bügel umfasst, der mit zwei Längsflügeln (36, 37) versehen ist, die dazu bestimmt sind, jeweils mit einer Abflachung (4a) des Fingerabstützrings (4) in Kontakt zu kommen.

4. Injektionsvorrichtung nach Anspruch 3, **dadurch gekennzeichnet, dass** der innere Arretierungsmechanismus des Kragens (9) der Kolbenstange (7) von transversalen Rillen (41) gebildet wird, die gegenüber den Flügeln (36, 37) des Bügels (35) angeordnet sind.

5. Injektionsvorrichtung nach Anspruch 3 oder 4, **dadurch gekennzeichnet, dass** der Bügel (35) einen Ring (38) umfasst, in dessen hinterer Verlängerung die Längsflügel (36, 37) verlaufen, wobei der innere Widerlagermechanismus des Fingerabstützrings (4) des Spritzenkörpers (1) eine geneigte Zunge (40) aufweist, die in Längsrichtung im Inneren des genannten Rings verläuft.

6. Injektionsvorrichtung nach einem der vorherigen Ansprüche, **dadurch gekennzeichnet, dass**:
- der hintere Teil (10) des Gehäuses außen die Form einer allgemein zylindrischen Manschette aufweist,
- der vordere Teil (11) des Gehäuses Abmessungen aufweist, die geeignet sind, in seiner vollen Länge im Inneren des hinteren Teils (10) des genannten Gehäuses aufgenommen zu werden,
- Führungsmechanismen (19, 25), die so gestaltet sind, dass sie eine Verschiebung über eine geringe Strecke des hinteren Teils (10) des Gehäuses entlang des vorderen Teils (11) des genannten Gehäuses zulassen.

7. Injektionsvorrichtung nach Anspruch 6, **dadurch gekennzeichnet, dass** die translationalen Arretierungsmittel der Haltemittel (35) des Kragens (9) der Kolbenstange (7) Folgendes umfassen:
- einen radial verformbaren Anschlagmechanismus (39) einstückig mit den Haltemitteln (35),
- einen axialen inneren Anschlagmechanismus (18) des verformbaren Anschlagmechanismus (39), der im Inneren des hinteren Teils (10) des Gehäuses angeordnet ist,
- einen'Drücker (15), der in der Umfangswand des hinteren Teils (10) des Gehäuses so angeordnet ist, dass er den Anschlagmechanismus (39) der Haltemittel (35) in der eingefahrenen Position der Letzteren verformen und freigeben kann,
- und einen Schlitz (31), der in der Umfangswand des vorderen Teils (11) des Gehäuses vorgesehen ist und eine Form aufweist, die so gestaltet ist, dass einerseits die Anschlagmechanismen (18, 39) durch den genannten Schlitz verlaufen und andererseits die Betätigung des Drückers (15) nur in der relativen ausgefahrenen Position der genannten Teile des Gehäuses möglich ist.

8. Injektionsvorrichtung nach Anspruch 7, **dadurch gekennzeichnet, dass** der Drücker (15) eine T-Längsform hat, der Schlitz (31) eine konjugierte Form aufweist und so angeordnet ist, dass eine Transversalverzweigung (32) entsteht, in Längsrichtung vom Transversalstab (15b) des Drückers (15) in der relativen eingefahrenen Position der Teile (10, 11) des Gehäuses versetzt ist.

9. Injektionsvorrichtung nach einem der Ansprüche 6 bis 8, **dadurch gekennzeichnet, dass** sie eine Verschlusskappe (43) mit einer Form umfasst, die so gestaltet ist, dass sie den Endabschnitt vor dem vorderen Teil (11) des Gehäuses verdeckt und am hinteren Teil (10) des Gehäuses in der eingefahrenen Position der Letzteren anschlägt.

10. Injektionsvorrichtung nach Anspruch 9, **dadurch gekennzeichnet, dass** der hintere Teil (10) des Gehäuses und die Verschlusskappe (43) profilierte Kontaktflächen (14, 45) in einer Nockenform aufweisen, die so gestaltet ist, dass sie den Rückzug der genannten Verschlusskappe durch eine Rotationsbewegung der Letzteren zulässt.

11. Injektionsvorrichtung nach einem der vorherigen Ansprüche, **dadurch gekennzeichnet, dass** der vordere Teil (11) des Gehäuses einen inneren Führungskanal (27) des Spritzenkörpers (1) aufweist, der auf einem Teil der Länge des genannten vorderen Teils ausgehend von der Frontwand (26) der Letzteren verläuft, wobei der genannte Kanal einen Widerlageransatz (27c) des genannten Spritzenkörpers in ihrer ausgefahrenen Position aufweist.

12. Injektionsvorrichtung nach Anspruch 11, **dadurch gekennzeichnet, dass** die Verschlusskappe (35) Greifer (48) aufweist, die in Längsrichtung im Inneren der genannten Verschlusskappe verlaufen und so angeordnet sind, dass sie im inneren Führungskanal (27) des Spritzenkörpers (1) aufgenommen werden, wobei die genannten Greifer so gestaltet sind, dass sie in einen Schutzansatz (5) der Nadel (3) der Spritze (1) einhaken.

## Claims

1. A device for automatically injecting a dose of a medicinal product, comprising:
- a long narrow casing having a front wall (26) pierced by an orifice (27b), and formed of two parts, a front part (11) and rear part (10) fitted one into the other and capable of sliding longitudinally in relation to each other over a short traverse so as to be able to undergo a relative movement between two positions : a retracted, so called rest position, of the rear part (10) with respect to the front part (11) obtained naturally in the absence of an external force exerted on the said parts, and a forward, so-called injection position, of the rear part (10) with respect to the front part (11) obtained by means of a longitudinal force exerted on the said rear part,
- a syringe (1) disposed in the casing and including a syringe body housing a piston (6), provided with a finger rest ring (4) and carrying a needle (3),
- a piston rod (7) provided with a collar (9) and extending into the rear extension of the syringe (1),
- means for supporting the collar (9) of the piston rod (7),
- a propulsion spring (50) extending between the means (35) for supporting the collar (9) of the piston rod (7) and the bottom of the rear part (10) of the casing,
- means (15, 18, 39) for locking in translation the means (35) for supporting the collar (9) of the piston rod (7), capable of supporting them inside the casing in a position where the propulsion spring (50) is compressed, and where the syringe (1) is entirely housed inside the said casing, and capable of allowing their movement towards a forward position at the end of injection, produced by the propulsion spring (50), after the parts (10, 11) of the casing are moved from their retracted position to their forward position,
- a device (27c) for stopping the syringe body (1) in a forward position of the latter where the needle (3) extends at least partially through the orifice (27b) of the casing,
- means (13) for triggering the means (35) for supporting the collar (9) of the piston rod (7), capable of freeing the said collar, in the forward position of the said supporting means, after the parts (10, 11) of the casing are moved from their forward position to their retracted position,
- and a return propulsion spring (51) with a force less than that of the propulsion spring (50), disposed around the syringe body (1) so as to be compressed by the finger rest ring (4) of the said syringe body when the latter is moved to its forward position,
the said injection device being **characterized in that**:
- it includes means for viewing (28, 29) provided in the wall of the casing so as to enable the means (35) for supporting the collar (9) of the piston rod (7) in their forward position to be seen,
- the means for supporting the collar (9) of the piston rod (7) comprise:
• an inner, so called guide tube (13) extending into the rear part (10) of the casing so as to delimit a peripheral annular space (21) within the latter adapted so as to house the propulsion spring (50) in the compressed position of the said spring,
• a cage (35) with a form adapted so as to slide inside the guide tube (13), and to house the piston rod (7) and the collar (9) thereof, the said cage including towards one of its ends, the so-called rear end, an internal device (41) for locking the collar (9) of the piston rod (7), capable of deforming radially and, towards its other forward end, a front wall (38) for stopping the propulsion spring (50) and for contacting the means (15, 18, 39) for locking in translation the said supporting means in the retracted position of the latter,
• the said guide tube and cage being adapted so that, in the forward position of the supporting means, the device (41) for locking the collar (9) of the piston rod (7) remains imprisoned in the guide tube (13) in the forward relative position of the parts (10, 11) of the casing, and is freed in the retracted relative position of the said parts of the casing.

2. The injection device as claimed in claim 1, **characterized in that** the guide tube (13) and the cage (35) have dimensions adapted so as to house the piston rod (7) and the finger rest ring (4) of the syringe body (1), the said cage including an internal device (40) for stopping the finger rest ring (4) of the syringe body (1) in the retracted position of the said cage, and a front wall (38) provided with an opening for permitting relative movement of this cage (35) and the syringe body (1) in the forward position of the latter.

3. The injection device as claimed in claim 2, **characterized in that** the finger rest ring (4) of the syringe body (1) has a truncated annular form and includes two diametrically opposed flats (4a), **characterized in that** the cage (35) includes a stirrup provided with two longitudinal legs (36, 37) arranged so that each comes into contact with a flat (4a) of the finger rest ring (4).

4. The injection device as claimed in claim 3, in which the internal device for locking the collar (9) of the piston rod (7) consists of transverse grooves (41) provided facing the legs (36, 37) of the stirrup (35).

5. The injection device as claimed in either of claims 3 or 4, **characterized in that** the stirrup (35) includes a ring (38) in the rear extension of which the longitudinal legs (36, 37) extend, the internal device for stopping the finger rest ring (4) of the syringe body (1) including at least one inclined tongue (40) extending longitudinally inside the said ring.

6. The injection device as claimed in one of the preceding claims, **characterized in that**:
- the rear part (10) of the casing has the form externally of a sleeve with a generally cylindrical form,
- the front part (11) of the casing has dimensions adapted so as to be housed over most of its length inside the rear part (10) of the said casing,
- the guiding devices (19, 25) are adapted so as to enable the rear part (10) of the casing to move over a short traverse along the front part (11) of the said casing.

7. The injection device as claimed in claim 6, **characterized in that** the means for locking in translation the means (35) for supporting the collar (9) of the piston rod (7) comprise:
- a radially deformable stop device (39) secured to the supporting means (35),
- an internal axial device (18) for stopping the deformable stop device (39), provided inside the rear part (10) of the casing,
- a trigger (15) provided in the peripheral wall of the rear part (10) of the casing so that it can deform and free the device (39) for stopping the supporting means (35), in the retracted position of the latter,
- and an opening (31) provided in the peripheral wall of the front part (11) of the casing and with a form adapted firstly so that the stop devices (18, 39) extend through the said opening and secondly so as to enable the trigger (15) to be actuated only in the forward relative position of the said parts of the casing.

8. The injection device as claimed in claim 7, **characterized in that** the trigger (15) has a longitudinal T-shape, the opening (31) having a corresponding shape and being provided so as to have a transverse arm (32) offset longitudinally from the transverse bar (15b) of the trigger (15) in the retracted relative position of the parts (10, 11) of the casing.

9. The injection device as claimed in one of claims 6 to 8, **characterized in that** it includes a cap (43) with a form adapted so as to close off the front end section of the front part (11) of the casing and to butt up against the rear part (10) of the said casing in the retracted position of the latter.

10. The injection device as claimed in claim 9, **characterized in that** the rear part (10) of the casing and the cap (43) have contact faces (14, 45) profiled in the shape of a cam so that the said cap can be removed by a rotational movement of the latter.

11. The injection device as claimed in one of the preceding claims, **characterized in that** the front part (11) of the casing incorporates an inner conduit (27) for guiding the syringe body (1) extending over a portion of the length of the said front part from the front wall (26) of the latter, the said conduit having a shoulder (27c) for stopping the said syringe body in its forward position.

12. The injection device as claimed in claim 11, **characterized in that** the cap (35) includes claws (48) extending longitudinally inside the said cap and disposed so as to lodge in the inner conduit (27) for guiding the syringe body (1), the said claws being adapted so as to hook over an end piece (5) protecting the needle (3) of the syringe (1).
